# EUROPÄISCHE PATENTSCHRIFT

(11) **EP 2 986 238 B1**
(45) Veröffentlichungstag und Bekanntmachung des Hinweises auf die Patenterteilung: **20.03.2019**
(21) Anmeldenummer: 13744471.7
(22) Anmeldetag: 22.07.2013
(51) Int. Cl.: A61B 17/28, A61B 17/29, A61B 17/00

(54) **ERGONOMISCHE SPERRMECHANIK**
ERGONOMIC LOCKING MECHANISM
MÉCANISME DE BLOCAGE ERGONOMIQUE

(30) Priorität: 20.04.2013 DE 102013006918
(43) Veröffentlichungstag der Anmeldung: 24.02.2016
(73) Patentinhaber: Aesculap AG, 78532 Tuttlingen (DE)
(72) Erfinder: STORZ, Martin, 78532 Tuttlingen (DE)
(74) Vertreter: Koller, Tobias Kilian
(86) Internationale Anmeldenummer: PCT/EP2013/065407
(87) Internationale Veröffentlichungsnummer: WO 2014/169970

(56) Entgegenhaltungen:
- WO-A2-2005/079680
- WO-A2-2009/132359
- DE-A1- 10 353 605
- DE-U1- 29 623 113
- DE-U1- 29 806 799
- US-A- 3 669 487
- US-A- 5 735 873
- US-A- 6 129 740
- US-A1- 2007 179 524
- US-A1- 2012 184 946

## Beschreibung

Die vorliegende Erfindung betrifft einen chirurgischen Instrumentengriff mit einer ergonomischen Sperrmechanik und insbesondere einen solchen Instrumentengriff für Rohrschaftinstrumente.

Chirurgische Instrumente verfügen je nach Griffart über eine Vielzahl unterschiedlicher Sperrmechaniken, deren Aufgabe es beispielsweise im Falle von Gefäßklemmen ist, das Festhalten des Gefäßes in Schließstellung zu übernehmen, sodass der Anwender das Instrument loslassen kann ohne die Sperrwirkung aufzugeben. Andererseits kann der Anwender jederzeit die Sperrmechanik lösen, das Instrument entnehmen oder es erneut an anderer Stelle zum Einsatz bringen. Instrumente mit Sperrmechaniken sind sowohl in der offenen als auch in der endoskopischen Chirurgie im Einsatz.

Im Falle von Ringbrancheninstrumenten in Kreuzhebelbauart, welche in der offenen Chirurgie die gängigste Instrumentenbauart darstellen, sind beidseitig zwischen den Branchen sich in Schließrichtung überrastende Zahnstangen angeordnet, welche sich ineinander verhaken und dadurch eine Sperrwirkung in Öffnungsrichtung bewirken. Das Lösen der Sperrwirkung erfolgt durch Verkanten der beiden Branchen, wobei die Einrastung ausgehoben wird. Nachteilig dabei ist, dass beim Lösen der Sperrdruck überwunden werden muss, wobei die sich in den Ringen befindlichen Finger gegen den Druck der Ringkanten eingesetzt werden müssen.

Im Falle von Schaftinstrumenten gehören ausschwenkbare Zahnstangen, welche am Ende der Branchen angebaut sind, zum Stand der Technik. Die Zahnstange gleitet unter leichtem Federdruck über einen feststehenden Widerhaken am gegenüberliegenden Branchenende, sodass eine Sperrwirkung in Öffnungsrichtung entsteht, welche durch Ausschwenken der Zahnstange gelöst werden kann.

Eine ergonomische Sperrmechanik an einem chirurgischen Instrumentengriff ist insbesondere für Rohrschaftinstrumente wichtig, da jede unergonomische Betätigung einer Sperrmechanik zu einer ungewollten Bewegung des Instruments führt. Aufgrund des relativ großen Abstands vom Griff zum Maulteil eines Rohrschaftinstruments wirkt sich bereits eine kleine ungewollte Verdrehung des Griffteils am Maulteil erheblich aus und führt zu einer deutlichen Bewegung desselben.

Im Stand der Technik sind zahlreiche Instrumentengriffe und Sperrmechaniken bekannt. Auch Instrumente mit sogenannten Pistolengriffen sind hinlänglich bekannt. Pistolengriffe sind Griffe, bei denen die Finger der Hand des Nutzers im Wesentlichen wie beim Halten einer Pistole gebeugt sind und sich der Rohrschaft oberhalb des Zeigefingers in etwa in die Richtung erstreckt, in die der ausgestreckte Zeigefinger zeigt. Zudem liegt der Daumen und/oder der Handballen an einem ersten Griffelement an und Zeige-, Mittel-, Ring- und kleiner Finger liegen an einem zweiten Griffelement an. Eines der beiden Griffelemente ist mit dem Rohrschaft starr verbunden und das andere Griffelement ist gelenkig mit dem Rohrschaft verbunden und zudem mit einem Betätigungsglied verbunden. Werden die beiden Griffelemente aufeinander zu bewegt, führt dies zu einer Relativbewegung von Rohrschaft und Betätigungsglied, welche im Maulteil in einen Öffnungs- bzw. Schließvorgang umgesetzt wird. Auf diese Weise kann das Maulteil mit dem Pistolengriff geöffnet und geschlossen werden oder in einer Position gehalten werden. Damit der Nutzer eine gewünschte Position nicht über längere Zeit halten muss, beispielsweise wenn ein Gewebestück gegriffen und gehalten werden soll, sind oft Sperrmechaniken an den Pistolengriffen vorgesehen. Diese schaffen eine lösbare Verbindung zwischen den beiden Griffelementen und sichern so deren gegenseitige Lage und damit die Stellung des Maulteils. Vergleichbare und weitere Instrumentengriffe sind zum Beispiel aus WO 12009/132359 A2, US2007/0179524 A1, DE298 06 799 U1, US 5,735,873 A1 und US 6,129,740 A1 bekannt.

WO 2005/079680 offenbart einen weiteren Instrumentengriff mit einem ersten Griffelement, verbunden mit einem Schaftbauteil, wobei eine Zeigefingerauflage und eine Handballenauflage am Griffelement ausgebildet sind, mit einem zweiten Griffelement mit Fingerauflagen, welches gelenkig verbunden ist mit dem ersten Griffelement und mit dem Betätigungsglied koppelbar ist, und mit einem am ersten Griffelement drehbar gelagerten Sperrelement, mit einem Betätigungsabschnitt und Sperrgliedern an beiden Griffelementen, das Sperrelement verhindert eine Vergrößerung des Abstandes zwischen dem ersten Griffelement und dem zweiten Griffelement wenn der Betätigungsabschnitt des Sperrelementes nicht aktiviert ist. In der DE 103 53 605 A1 ist ein solcher Pistolengriff mit einer Sperrmechanik gezeigt. Die Sperrmechanik ist durch den Zeigefinger betätigbar, d.h. lösbar, indem der Zeigefinger zu dem vorderen Griffelement hin bewegt wird und dabei ein Sperrelement betätigt. Das Sperrelement verfügt über einen Betätigungsabschnitt und ein Sperrglied, wobei das Sperrelement zwischen Betätigungsabschnitt und Sperrglied drehbar an einem Vorsprung gelagert ist, der sich von dem vorderen Bereich des hinteren Griffelements nach unten erstreckt. Eine Blattfeder drängt dabei den Betätigungsabschnitt von dem vorderen Griffelement nach proximal, also zu dem Maulteil hin. An dem Sperrglied, welches gebogen ausgebildet ist, ist ein kreisbogenförmiger gezahnter Abschnitt vorgesehen, dessen Zentrum die gelenkige Verbindung zwischen den beiden Griffelementen ist und der mit einem Sperrzahn zusammenwirkt, welcher starr an dem vorderen Griffelement ausgebildet ist.

Der vorstehend beschriebene chirurgische Instrumentengriff mit Sperrmechanik hat folgende zwei Nachteile. Zum einen muss, um die Sperrwirkung der Sperrmechanik zu lösen, der Zeigefinger zu dem vorderen Griffelement hin bewegt werden und muss den Betätigungsabschnitt gegen die Kraft der Blattfeder zu dem vorderen Griffelement hin gedrückt werden, also nach distal. Gleichzeitig muss das vordere Griffelement nach proximal bewegt werden. Der Zeigefinger muss also eine Bewegung ausführen, die gegensätzlich zu der Bewegung von Ring-, Mittel- und kleinem Finger ist. Dies ist keine natürliche Bewegung und damit unergonomisch und führt leicht zu einer ungewollten Bewegung des Instruments. Wenn der Zeigefinger den Betätigungsabschnitt der Sperrmechanik nach proximal drückt, liegt er auch an der Zeigefingerauflage an, welche geschlitzt ist und in der der Betätigungsabschnitt angeordnet ist. In diesem Fall wird das Instrument durch Zeigefinger und Daumen bzw. Handballen sicher gehalten. In dem Moment, in dem die Sperrmechanik in Eingriff gebracht wird, indem der Zeigefinger von dem Betätigungsabschnitt und somit auch von der Zeigefingerauflage weg bewegt wird, wird das Instrument nicht mehr sicher gehalten, da nun nur noch Daumen bzw. Handballen und Ring-, Mittel und kleiner Finger das Halten bewirken und die drei letztgenannten Finger nur an dem gelenkig gelagerten vorderen Griffelement anliegen. In diesem Zustand kann leicht eine ungewollte Bewegung des Instruments verursacht werden. Es ist somit die Aufgabe der vorliegenden Erfindung, einen chirurgischen Instrumentengriff zu schaffen, der zu jedem Zeitpunkt sicher gehalten werden kann und ein ergonomisches Aktivieren bzw. Lösen der Sperrmechanik erlaubt. Eine weitere Aufgabe der vorliegenden Erfindung ist es eine solche Sperrmechanik bereit zu stellen, bei der während einem Öffnungs- und Schließvorgang kein Ratschen auftritt, d.h. bei der der Sperrzahn nicht über den gezahnten Abschnitt gleitet.

Die Aufgabe der vorliegenden Erfindung wird durch einen chirurgischen Instrumentengriff nach Anspruch 1 und ein chirurgisches Instrument mit einem solchen Griff nach Anspruch 9 gelöst. Vorteilhafte Ausbildungen und Weiterentwicklungen sind Gegenstand der abhängigen Ansprüche.

Ein chirurgischer Instrumentengriff gemäß einem ersten Aspekt der vorliegenden Erfindung, der an einem chirurgischen Instrument zum Einsatz kommt, weist Folgendes auf: ein erstes Griffelement, welches mit einem von einem Schaftbauteil und einem Betätigungsglied eines chirurgischen Instruments starr verbindbar ist, wobei an dem ersten Griffelement eine Zeigefingerauflage und eine Handballenauflage ausgebildet sind. Zudem weist es ein zweites Griffelement auf, welches gelenkig mit dem ersten Griffelement verbunden ist und mit dem anderen von dem Schaftbauteil und dem Betätigungsglied des chirurgischen Instruments koppelbar ist, wobei an dem zweiten Griffelement mindestens eine weitere Fingerauflage ausgebildet ist, wobei das erste Griffelement daran angepasst ist, von einem Zeigefinger und einem Handballen einer Hand gegriffen und gehalten zu werden. An dem ersten Griffelement ist zudem ein Sperrelement drehbar angebracht, wobei das Sperrelement einen Betätigungsabschnitt und ein erstes Sperrglied aufweist. An dem zweiten Griffelement ist ein zweites Sperrglied vorgesehen und das erste Sperrglied ist elastisch zu dem zweiten Sperrglied hin vorgespannt. Das erste und das zweite Sperrglied sind daran angepasst, zusammenzuwirken, um eine Vergrößerung des Abstands zwischen der Handballenauflage an dem ersten Griffelement und der mindestens einen weiteren Fingerauflage an dem zweiten Griffelement zu verhindern. Darüber hinaus weist der Betätigungsabschnitt eine Daumenauflage auf, die daran angepasst ist, den Daumen eines Nutzers im Wesentlichen von unten zu stützen.

Mit einem solchen chirurgischen Instrumentengriff ist eine hervorragend ergonomische Handhabung der Sperre möglich. Gleichzeitig ist das Instrument sehr gut greifbar und dadurch präzise positionierbar und steuerbar. Dies wird insbesondere dadurch erzielt, dass das erste Griffelement mit Zeigefinger und Handballen gegriffen wird. Diese beiden Extremitäten haben an einer Hand einen relativ großen Abstand zueinander, sodass der Griffhebel deutlich größer als bei anderen Griffdesigns ist. Auf diese Weise kann der Griff fester gehalten werden und es treten deutlich geringere ungewollte Verdrehungen des Instruments in der Hand auf.

Darüber hinaus ist die Bedienung der Sperrmechanik durch eine Bewegung des Daumens der Hand des Nutzers, mit der er den Griff hält, im Wesentlichen nach unten oder nach oben möglich. Diese Bewegung ist also keiner Bewegungsrichtung eines anderen Fingers der haltenden Hand entgegengesetzt oder interagiert mit irgend einer anderen Bewegung der Hand oder der Finger des Nutzers. Wird die Daumenauflage nach unten gedrückt, so rückt das erste Sperrglied aus dem zweiten Sperrglied aus und der Instrumentengriff kann leicht geöffnet werden. Wird die Daumenauflage nach oben gedrückt - sei es durch den Daumen selbst oder durch ein elastisches Element, welches die Daumenauflage in ihre Ausgangsposition drängt - rückt das zweite Sperrglied in das erste Sperrglied ein und verhindert ein Öffnen des Instrumentengriffs.

Gemäß einer vorteilhaften Ausgestaltung des ersten Aspekts der vorliegenden Erfindung ist das Sperrelement zwischen dem Betätigungsabschnitt und dem ersten Sperrglied an dem ersten Griffelement drehbar angebracht. Mit dieser Ausbildung kann eine besonders ergonomische Anordnung des Betätigungsabschnitts erreicht werden, ohne dass die Sperrglieder zu nahe an dem Drehpunkt der beiden Griffelemente zueinander angeordnet sein muss. Alternativ dazu können der Dreh- und Montagepunkt, der Betätigungsabschnitt mitsamt Daumenauflage und das erste Sperrglied in einer Linie ausgebildet sein. In diesem Fall könnte aber der Daumen des Nutzers mit dem zweiten Sperrglied in Berührung kommen, was durch den Nutzer zumindest als unangenehm empfunden würde und was auch eine echte Behinderung des Öffnungs- bzw. Schließvorgangs des Griffs darstellt. Zudem müsste die Bewegung des Daumens sehr fein gesteuert werden, da dieser Aufbau über die unterschiedlichen Hebellängen zu einer Verstärkung (bzw. Vergrößerung) der Bewegung des ersten Sperrglieds führt.

Gemäß noch einer vorteilhaften Ausgestaltung des ersten Aspekts der vorliegenden Erfindung ist ein elastisches Element zwischen dem ersten Griffelement und dem zweiten Griffelement angeordnet, welches daran angepasst ist, die Griffelemente in eine Öffnungsstellung zu drängen. Dabei ist das elastische Element bevorzugt eine Blattfeder, die weiter bevorzugt an dem ersten Griffteil befestigt ist und an dem zweiten Griffelement anliegt. Mit diesem Aufbau muss ein Öffnen des Griff nicht aktiv durch Mittel-, Ring- und kleinen Finger bewirkt werden, sondern der Griff öffnet sich automatisch bei nachlassender Schließkraft, die durch die vorgenannten Finger aufgebracht wird. Für ein durch die genannten Finger aktiv betriebenes Öffnen des Griffs wäre zumindest eine Anlagefläche für wenigstens einen der Finger erforderlich. In diesem Fall könnte zumindest eine der Anlageflächen für wenigstens einen der Finger im Wesentlichen ringförmig ausgebildet sein. oder zwischen zwei Fingern ist ein Vorsprung ausgebildet, der eine solche Anlagefläche bildet.

Gemäß einer weiteren vorteilhaften Ausgestaltung des ersten Aspekts der vorliegenden Erfindung ist die Zeigefingerauflage, die Handballenauflage und/oder wenigstens eine weitere Fingerauflage als im Wesentlichen geschlossener Ring ausgebildet. Mit einer solchen Ausgestaltung kann der Griff zum einen auch geöffnet werden, wenn keine Feder die beiden Griffelemente in Öffnungsrichtung drängt, und zum anderen bildet diese Art der Fingerauflage eine Art Schutzbügel für die Finger.

Gemäß noch einer weiteren vorteilhaften Ausgestaltung des ersten Aspekts der vorliegenden Erfindung sind die Zeigefingerauflage und die Handballenauflage mit einem Steg verbunden und die Daumenauflage ist zumindest in der Ausgangsstellung oberhalb des Steges angeordnet. Mit einer solchen Ausgestaltung entsteht ein besonders schlanker und leichter Instrumentengriff. Zudem ist so ausreichend Platz an dem Instrumentengriff um die Daumenauflage besonders ergonomisch anzuordnen und gleichzeitig die Sperrglieder optimal aneinander anzupassen und so an dem Griff zu platzieren, dass sie bei der Betätigung nicht stören.

Gemäß einer anderen vorteilhaften Ausgestaltung des ersten Aspekts der vorliegenden Erfindung weist das erste Sperrglied einen Sperrzahn auf, der zu dem zweiten Sperrglied hin vorsteht, und das zweite Sperrglied weist eine Zahnstange auf, die dem ersten Sperrglied zugewandt ist und die bevorzugt kreisbogenförmig gestaltet ist. Auf diese Weise kann ein definierter Eingriff des Sperrzahns in die Zahnstange erfolgen. Die bevorzugte Kreisbogenform der Zahnstange bewirkt, dass die Bewegung des Sperrelements zum Einrücken bzw. Ausrücken des Sperrzahns in bzw. aus der Zahnstange in jeder Schließposition der Griffelemente zueinander identisch ist. Die von dem Instrument eingenommene Schließposition ist nicht immer identisch und hängt bei einer Fasszange z.B. von dem gegriffenen Gewebe oder Element bzw. dessen Dicke ab. Wenn die erforderliche Bewegung des Sperrelements und dabei vor allem des Betätigungsabschnitts mit der Daumenauflage in jeder der möglichen Schließpositionen immer identisch ist, führt dies zu einer besonders sicheren Handhabung des Instrumentengriffs.

Gemäß wieder einer anderen vorteilhaften Ausgestaltung des ersten Aspekts der vorliegenden Erfindung ist das erste Sperrglied mittels einer Blattfeder zu dem zweiten Sperrglied hin vorgespannt, welche an dem ersten Griffelement befestigt ist und an dem Sperrelement anliegt.

Gemäß wieder einer weiteren vorteilhaften Ausgestaltung des ersten Aspekts der vorliegenden Erfindung erstreckt sich die Daumenauflage beiderseits der Längsachse des ersten Griffelements 10, um rechtshändig und linkshändig betätigbar zu sein. Auf diese Weise muss kein gesonderter Instrumentengriff für Rechtshänder und Linkshänder bereit gestellt werden.

Gemäß einem zweiten Aspekt der vorliegenden Erfindung ist ein chirurgisches Instrument offenbart mit einem Schaftbauteil, einem Betätigungsglied, und einem Maulteil, welches durch eine axiale Relativbewegung von Schaftbauteil und Betätigungsglied betätigbar ist. Das chirurgische Instrument weist dabei einen chirurgischen Instrumentengriff nach einem der vorstehenden Ansprüche auf. Mit einem solchen chirurgischen Instrument, bei dem der vorstehend beschriebene chirurgische Instrumentengriff fest oder lösbar verbaut ist, lassen sich die vorstehend beschriebenen Vorteile erzielen.

Gemäß einer vorteilhaften Ausgestaltung des zweiten Aspekts der vorliegenden Erfindung ist das erste Griffelement starr mit einem von einem Schaftbauteil und einem Betätigungsglied eines chirurgischen Instruments verbunden und das zweite Griffelement ist mit dem anderen von dem Schaftbauteil und dem Betätigungsglied des chirurgischen Instruments gekoppelt. Auf diese Weise wird ein Instrument geschaffen, welches besonders präzise handhabbar ist, da eine starre Verbindung zumeist steifer als eine lösbare Verbindung ist.

Weitere Vorteile und Merkmale der Erfindung sind dem Fachmann aus den beigefügten Figuren und der detaillierten Beschreibung der Ausführungsbeispiele ersichtlich.
- Fig. 1: zeigt eine seitliche Ansicht eines chirurgischen Instrumentengriffs gemäß einem ersten Ausführungsbeispiel in einer Hand eines Nutzers;
- Fig. 2: zeigt eine Ansicht entsprechend Fig. 1 ohne die Hand eines Nutzers;
- Fig. 3A: zeigt eine seitliche Ansicht eines chirurgischen Instrumentengriffs gemäß einem zweiten Ausführungsbeispiel;
- Fig. 3B: zeigt eine Ansicht des chirurgischen Instrumentengriffs entsprechend Fig. 3A von oben;
- Fig. 4A: zeigt eine seitliche Ansicht eines chirurgischen Instrumentengriffs gemäß einem dritten Ausführungsbeispiel; und
- Fig. 4B: zeigt eine Ansicht des chirurgischen Instrumentengriffs entsprechend Fig. 4A von oben.

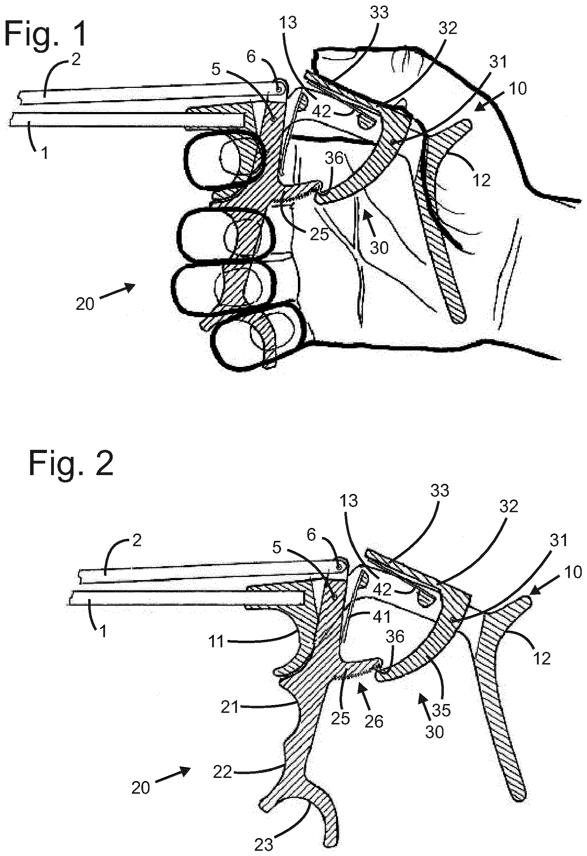

Ein erstes Ausführungsbeispiel der vorliegenden Erfindung ist unter Bezugnahme auf die Fig. 1 und 2 im Detail beschrieben.

Der chirurgische Instrumentengriff dieses Ausführungsbeispiels ist für ein nicht näher beschriebenes chirurgisches Instrument geschaffen, beispielsweise eine endoskopische Fasszange. Schaftbauteil 1 und Betätigungsglied 2 sind nur schematisch gezeigt und das Betätigungsglied 2 kann durchaus in einem Rohrschaftbauteil 1 verlaufen. Um eine Bewegung des Betätigungsglieds 2 nach oben und unten während der Betätigung des Instrumentengriffs zu vermeiden kann das Betätigungsglied mit einem Stift an einem Langloch verankert sein, welches an Stelle der Gelenkverbindung 6 in dem zweiten Griffelement ausgebildet ist.

Der Instrumentengriff weist ein erstes Griffelement 10 auf, welches mit dem Schaftbauteil 1 starr verbunden ist. An dem ersten Griffelement 10 sind eine Zeigefingerauflage 11 und eine Handballenauflage 12 ausgebildet, in die der Zeigefinger bzw. der Handballen des Nutzers eingelegt wird. Die beiden Auflagen 11 und 12 sind mit einem Steg 13 verbunden. Ein zweites Griffelement 20, welches an einem Gelenkpunkt 5 mit dem ersten Griffelement 10 verbunden ist, ist mit dem Betätigungsglied 2 des chirurgischen Instruments an dem Gelenk 6 gekoppelt. An dem zweiten Griffelement 20 sind drei einzelne Fingerauflagen 21, 22, 23 ausgebildet. An dem ersten Griffelement 10 ist ein Sperrelement 30 drehbar angebracht, welches einen Betätigungsabschnitt 32 und ein erstes Sperrglied 35 aufweist. Das erste Sperrglied ist ein einzelner Sperrzahn 36. An dem zweiten Griffelement 20 ist ein zweites Sperrglied 25 in Form einer Zahnstange 26 vorgesehen, deren Seite, die dem Sperrzahn 36 zugewandt ist, kreisbogenförmig ist. Das erste Sperrglied 35 ist mittels einer Blattfeder 42 elastisch zu dem zweiten Sperrglied 25 hin vorgespannt. Die Blattfeder 42 ist an dem Steg 13 des ersten Griffelements 10 befestigt und liegt im Wesentlichen von unten an einer Daumenauflage 33 an, die an dem Betätigungsabschnitt 32 des Sperrelements 30 ausgebildet ist.

Der Sperrzahn 36 und die Zahnstange 26 sind daran angepasst, zusammenzuwirken, um eine Vergrößerung des Abstands zwischen der Handballenauflage 12 an dem ersten Griffelement 10 und den Fingerauflagen 21, 22, 23 an dem zweiten Griffelement 20 zu verhindern. Dazu kann der Sperrzahn 36 in die Zahnzwischenräume der Zahnstange 26 eintreten. Durch die Form des Sperrzahns 36 und der Zähne der Zahnstange 26 kann es ermöglicht werden, dass der Sperrzahn 36 in Schließrichtung über die Zahnstange 26 hinweg gleiten kann, ohne dass das Sperrelement 30 betätigt werden bzw. sein muss, wohingegen eine Bewegung in der Gegenrichtung ohne eine Betätigung des Sperrelements 30 sicher verhindert ist. Entsprechende Ausformungen von Zahnstange 26 und Sperrzahn 36 sind im Stand der Technik bekannt. Die Daumenauflage 33 ist so gestaltet, dass sie den Daumen eines Nutzers im Wesentlichen von unten stützt, wie dies in Fig. 1 gezeigt ist. Zudem befindet sich die Daumenauflage 33 bei diesem Ausführungsbeispiel stets oberhalb des Stegs 13 und erstreckt sich lateral beiderseits des Stegs 13, sodass der Instrumentengriff für Rechtshänder und Linkshänder benutzbar ist.

Der Punkt 31, an dem das Sperrelement 30 an dem Steg 13 des ersten Griffelements 10 gelenkig befestigt ist, befindet sich zwischen dem Betätigungsabschnitt 32 mit der Daumenauflage 33 und dem ersten Sperrglied 35 mit dem Sperrzahn 36. Eine Blattfeder 41 ist zwischen dem ersten Griffelement 10 und dem zweiten Griffelement 20 angeordnet und drängt die Griffelemente 10, 20 in eine Öffnungsstellung. Die Blattfeder 41 ist an dem ersten Griffelement 10 befestigt und liegt an dem zweiten Griffelement 20 an.

Indem der Daumen eines Nutzers leicht gebeugt wird, bewegt sich die Daumenkuppe, welche sich bei einer ergonomischen Handhaltung auf der Daumenauflage 33 befindet, nach unten. Dadurch wird das Sperrelement 30 um den Gelenkpunkt 31 gedreht und der Sperrzahn 36 des ersten Sperrglieds 35 bewegt sich außer Eingriff mit den Zähnen der Zahnstange 26 des zweiten Sperrglieds 25, welches einstückig mit dem zweiten Griffelement 20 ausgebildet ist und zu dem ersten Griffelement 10 hin vorsteht. In diesem Zustand kann ein beliebiges Öffnen und Schließen des Instrumentengriffs und somit des zugehörigen Mauls des Instruments bewirkt werden. Um eine Schließposition zu sperren, d.h. ein ungewolltes Öffnen aus dieser Position heraus zu verhindern, streckt der Nutzer den Daumen so weit aus, bis die Daumenauflage 33 und somit das Sperrglied 30 in seine Ausgangslage zurück kehrt. Dabei tritt der Sperrzahn 36 in Eingriff mit der Zahnstange 26 und ein Sperren des Instrumentengriffs wird bewirkt.

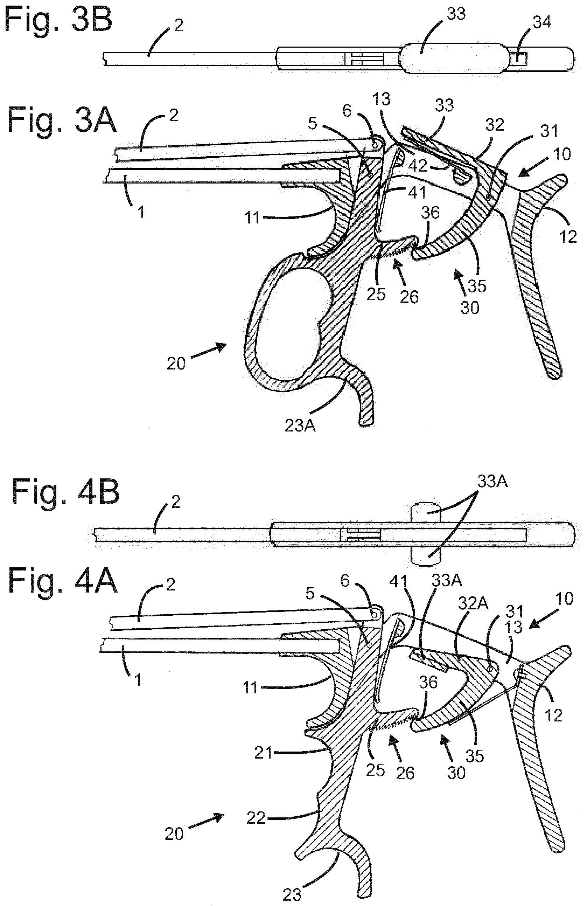

Im Folgenden ist ein zweites Ausführungsbeispiel der vorliegenden Erfindung unter Bezugnahme auf die Fig. 3A und 3B beschrieben, wobei hier vorwiegend die Unterschiede zu dem ersten Ausführungsbeispiel erläutert sind. Die einzigen Unterschiede zu dem ersten Ausführungsbeispiel liegen darin, dass die Daumenauflage 33 relativ schmal ausgebildet ist und dass die Fingerauflagen für den Mittelfinger und den Ringfinger als eine gemeinsame ringförmige Auflage 21A ausgebildet sind. Wie dies in Fig. 3B gezeigt ist, ist der Steg 13 des ersten Griffelements 10 geschlitzt und das zweite Griffelement 20 sowie das Sperrelement 30 sind in diesem Schlitz 34 angeordnet. Auf diese Weise weist das erste Griffelement 10 quasi 2 Stege auf. Prinzipiell kann das erste Griffelement 10 aber auch nur einen Steg 13 aufweisen und das zweite Griffelement 20 sowie das Sperrglied 30 werden nicht in einem Schlitz 34 angeordnet sondern seitlich des dann einzigen Stegs 13.

Ein drittes Ausführungsbeispiel der vorliegenden Erfindung ist im Folgenden im Detail unter Bezugnahme auf die Fig. 4A und 4B beschrieben, wobei wiederum vorwiegend die Unterschiede zum ersten Ausführungsbeispiele erläutert sind. Das Sperrelement 30A weist eine sehr breite Daumenauflage 33B auf, welche zudem unterhalb des Stegs 13 angeordnet ist. Die Feder 42A, mit der das Sperrelement 30A in die Sperrposition gedrängt wird, ist im Bereich der Handballenauflage 12 befestigt und liegt von schräg unten an dem ersten Sperrglied 35 an. Die Daumenballenauflage 33B ist deshalb so breit gestaltet, da sich der Daumen in der Nutzungsposition nun nicht über dem Steg 13 sondern neben dem Steg 13 befindet. Um ein versehentliches Abrutschen des Daumens von der Daumenauflage 33B zu verhindern, ist diese entsprechend zu dimensionieren.

Dem Fachmann ergeben sich zudem aus den anliegenden Ansprüchen, der obigen Beschreibung und den Figuren zahlreiche weitere Ausführungsformen und Abwandlungen der vorliegenden Erfindung. Selbstverständlich können einzelne Merkmale oder Gruppen von Merkmalen der verschiedenen Ausführungsbeispiele beliebig geeignet miteinander kombiniert werden. Die Blattfedern können beispielsweise auch durch Schenkelfedern oder Torsionsfedern substituiert werden.

## Patentansprüche

1. Chirurgischer Instrumentengriff für ein chirurgisches Instrument mit
einem ersten Griffelement (10), welches mit einem von einem Schaftbauteil (1) und einem Betätigungsglied (2) eines chirurgischen Instruments starr verbindbar ist, wobei an dem ersten Griffelement (10) eine Zeigefingerauflage (11) und eine Handballenauflage (12) ausgebildet sind,
einem zweiten Griffelement (20), welches gelenkig mit dem ersten Griffelement (10) verbunden ist und mit dem anderen von dem Schaftbauteil (1) und dem Betätigungsglied (2) des chirurgischen Instruments koppelbar ist, wobei an dem zweiten Griffelement (20) mindestens eine weitere Fingerauflage (21, 22, 23; 21A, 23A) ausgebildet ist, wobei das erste Griffelement (10) daran angepasst ist, von einem Zeigefinger und einem Handballen einer Hand gegriffen und gehalten zu werden,
an dem ersten Griffelement (10) ein Sperrelement (30) drehbar angebracht ist, wobei das Sperrelement (30) einen Betätigungsabschnitt (32, 32A) und ein erstes Sperrglied (35) aufweist,
an dem zweiten Griffelement (20) ein zweites Sperrglied (25) vorgesehen ist,
das erste Sperrglied (35) elastisch zu dem zweiten Sperrglied (25) hin vorgespannt ist,
das erste und das zweite Sperrglied (25, 35) daran angepasst sind, zusammenzuwirken, um eine Vergrößerung des Abstands zwischen der Handballenauflage (12) an dem ersten Griffelement (10) und der mindestens einen weiteren Fingerauflage (21, 22, 23; 21A, 23A) an dem zweiten Griffelement (20) zu verhindern,
**dadurch gekennzeichnet, dass** der Betätigungsabschnitt (32, 32A) eine Daumenauflage (33, 33A) aufweist, die daran angepasst ist, den Daumen eines Nutzers im Wesentlichen von unten zu stützen.

2. Chirurgischer Instrumentengriff nach Anspruch 1, wobei das Sperrelement (30) zwischen dem Betätigungsabschnitt (32) und dem ersten Sperrglied (35) an dem ersten Griffelement (10) drehbar angebracht ist.

3. Chirurgischer Instrumentengriff nach Anspruch 1 oder 2, wobei
ein elastisches Element (41) zwischen dem ersten Griffelement (10) und dem zweiten Griffelement (20) angeordnet ist, welches daran angepasst ist, die Griffelemente (10, 20) in eine Öffnungsstellung zu drängen, wobei das elastische Element (41) bevorzugt eine Blattfeder ist, die weiter bevorzugt an dem ersten Griffteil (10) befestigt ist und an dem zweiten Griffelement (20) anliegt.

4. Chirurgischer Instrumentengriff nach einem der Ansprüche 1 bis 3, wobei
die Zeigefingerauflage (11), die Handballenauflage (12) und/oder wenigstens eine weitere Fingerauflage (21, 22, 23, 21A, 23A) als im Wesentlichen geschlossener Ring (21A) ausgebildet ist.

5. Chirurgischer Instrumentengriff nach einem der Ansprüche 1 bis 4, wobei
die Zeigefingerauflage (11) und die Handballenauflage (12) mit einem Steg (13) verbunden sind und die Daumenauflage (33) zumindest in der Ausgangsstellung oberhalb des Steges (13) angeordnet ist.

6. Chirurgischer Instrumentengriff nach einem der Ansprüche 1 bis 5, wobei
das erste Sperrglied (35) einen Sperrzahn (36) aufweist, der zu dem zweiten Sperrglied (25) hin vorsteht, und
das zweite Sperrglied (25) eine Zahnstange (26) aufweist, die dem ersten Sperrglied (35) zugewandt ist und die bevorzugt kreisbogenförmig gestaltet ist.

7. Chirurgischer Instrumentengriff nach einem der Ansprüche 1 bis 6, wobei
das erste Sperrglied (35) mittels einer Blattfeder (42) zu dem zweiten Sperrglied (25) hin vorgespannt ist, die bevorzugt an dem ersten Griffelement (10) befestigt ist und an dem Sperrelement (30) anliegt.

8. Chirurgischer Instrumentengriff nach einem der Ansprüche 1 bis 7, wobei
die Daumenauflage (33, 33A) sich beiderseits der Längsachse des ersten Griffelements (10) erstreckt, um rechtshändig und linkshändig betätigbar zu sein.

9. Chirurgisches Instrument mit
einem Schaftbauteil (1),
einem Betätigungsglied (2),
einem Maulteil, welches durch eine axiale Relativbewegung von Schaftbauteil (1) und Betätigungsglied (2) betätigbar ist,
**gekennzeichnet durch**
einen chirurgischen Instrumentengriff nach einem der vorstehenden Ansprüche.

10. Chirurgisches Instrument nach Anspruch 9, wobei
das erste Griffelement (10) starr mit einem von einem Schaftbauteil (1) und einem Betätigungsglied (2) eines chirurgischen Instruments verbunden ist, und
das zweite Griffelement (20) mit dem anderen von dem Schaftbauteil (1) und dem Betätigungsglied (2) des chirurgischen Instruments gekoppelt ist.

## Claims

1. Surgical instrument grip for a surgical instrument, having
a first grip element (10), which is rigidly connectable to one of a shank component (1) and an actuating member (2) of a surgical instrument, wherein an index finger support (11) and a support (12) for the heel of the hand are formed on the first grip element (10),
a second grip element (20), which is connected to the first grip element (10) in an articulated manner and is able to be coupled to the other of the shank component (1) and the actuating member (2) of the surgical instrument, wherein at least one further finger support (21, 22, 23; 21A, 23A) is formed on the second grip element,
wherein
the first grip element (10) is designed to be gripped and held by an index finger and a heel of a hand,
a locking element (30) is rotatably attached to the first grip element (10), wherein the locking element (30) has an actuating portion (32, 32A) and a first locking member (35),
a second locking member (25) is provided on the second grip element (20),
the first locking member (35) is preloaded elastically towards the second locking member (25),
the first and the second locking member (25, 35) are designed to cooperate in order to prevent an increase in the distance between the support (12) for the heel of the hand on the first grip element (10) and the at least one further finger support (21, 22, 23; 21A, 23A) on the second grip element (20),
**characterized in that**
the actuating portion (32, 32A) has a thumb support (33, 33A) which is designed to support a user's thumb substantially from below.

2. Surgical instrument grip according to Claim 1, wherein the locking element (30) is rotatably attached to the first grip element (10) between the actuating portion (32) and the first locking member (35).

3. Surgical instrument grip according to Claim 1 or 2, wherein
an elastic element (41) is arranged between the first grip element (10) and the second grip element (20), which is designed to urge the grip elements (10, 20) into an open position, wherein the elastic element (41) is preferably a leaf spring, which is more preferably fastened to the first grip part (10) and bears against the second grip element (20).

4. Surgical instrument grip according to one of Claims 1 to 3, wherein
the index finger support (11), the support (12) for the heel of the hand and/or at least one further finger support (21, 22, 23; 21A, 23A) is configured as a substantially closed ring (21A).

5. Surgical instrument grip according to one of Claims 1 to 4, wherein
the index finger support (11) and the support (12) for the heel of the hand are connected by a bar (13) and the thumb support (33) is arranged above the bar (13) at least in the starting position.

6. Surgical instrument grip according to one of Claims 1 to 5, wherein
the first locking member (35) has a locking tooth (36) that protrudes towards the second locking member (25), and
the second locking member (25) has a toothed bar (26) that faces the first locking member (35) and is preferably in the shape of a circular arc.

7. Surgical instrument grip according to one of Claims 1 to 6, wherein
the first locking member (35) is preloaded towards the second locking member (25) by means of a leaf spring (42), which is preferably fastened to the first grip element (10) and bears against the locking element (30).

8. Surgical instrument grip according to one of Claims 1 to 7, wherein
the thumb support (33, 33A) extends on both sides of the longitudinal axis of the first grip element (10) in order to be actuable in a right-handed and a left-handed manner.

9. Surgical instrument having
a shank component (1),
an actuating member (2),
a jaw part that is actuable by an axial relative movement of the shank component (1) and actuating member (2),
**characterized by**
a surgical instrument grip according to one of the preceding claims.

10. Surgical instrument according to Claim 9, wherein
the first grip element (10) is rigidly connected to one of a shank component (1) and an actuating member (2) of a surgical instrument, and
the second grip element (20) is coupled to the other of the shank component (1) and the actuating member (2) of the surgical instrument.

## Revendications

1. Poignée d'instrument chirurgical pour un instrument chirurgical comprenant
un premier élément de poignée (10) qui peut être connecté rigidement à l'un parmi un composant de tige (1) ou un organe d'actionnement (2) d'un instrument chirurgical, un appui pour l'index (11) et un appui pour la paume de la main (12) étant réalisés sur le premier élément de poignée (10),
un deuxième élément de poignée (20) qui est connecté de manière articulée au premier élément de poignée (10) et qui peut être accouplé à l'autre parmi le composant de tige (1) ou l'organe d'actionnement (2) de l'instrument chirurgical, au moins un appui supplémentaire pour les doigts (21, 22, 23 ; 21A, 23A) étant réalisé sur le deuxième élément de poignée (20),
le premier élément de poignée (10) étant adapté de manière à pouvoir être saisi et tenu par un index et une paume d'une main,
un élément de blocage (30) étant monté de manière rotative sur le premier élément de poignée (10), l'élément de blocage (30) présentant une portion d'actionnement (32, 32A) et un premier organe de blocage (35),
un deuxième organe de blocage (25) étant prévu sur le deuxième élément de poignée (20),
le premier organe de blocage (35) étant précontraint élastiquement vers le deuxième organe de blocage (25),
le premier et le deuxième organe de blocage (25, 35) étant adaptés de manière à coopérer, afin d'empêcher une augmentation de la distance entre l'appui pour la paume de la main (12) sur le premier élément de poignée (10) et l'au moins un appui supplémentaire pour les doigts (21, 22, 23 ; 21A, 23A) sur le deuxième élément de poignée (20), **caractérisée en ce que**
la portion d'actionnement (32, 32A) présente un appui pour le pouce (33, 33A) qui est adapté pour supporter sensiblement par le dessous le pouce d'un utilisateur.

2. Poignée d'instrument chirurgical selon la revendication 1, dans laquelle l'élément de blocage (30) est monté de manière rotative entre la portion d'actionnement (32) et le premier organe de blocage (35) sur le premier élément de poignée (10).

3. Poignée d'instrument chirurgical selon la revendication 1 ou 2, dans laquelle un élément élastique (41) est disposé entre le premier élément de poignée (10) et le deuxième élément de poignée (20), lequel est adapté de manière à presser les éléments de poignée (10, 20) dans une position d'ouverture, l'élément élastique (41) étant de préférence un ressort à lame qui est de préférence en outre fixé à la première partie de poignée (10) et qui s'applique contre le deuxième élément de poignée (20).

4. Poignée d'instrument chirurgical selon l'une quelconque des revendications 1 à 3, dans laquelle l'appui pour l'index (11), l'appui pour la paume de la main (12) et/ou au moins un appui supplémentaire pour les doigts (21, 22, 23 ; 21A, 23A) sont réalisés sous forme de bague essentiellement fermée (21A).

5. Poignée d'instrument chirurgical selon l'une quelconque des revendications 1 à 4, dans laquelle l'appui pour l'index (11) et l'appui pour la paume de la main (12) sont reliés par une tige (13) et l'appui pour le pouce (33) est disposé au moins dans la position de départ au-dessus de la tige (13) .

6. Poignée d'instrument chirurgical selon l'une quelconque des revendications 1 à 5, dans laquelle le premier organe de blocage (35) présente une dent de blocage (36) qui fait saillie vers le deuxième organe de blocage (25), et
le deuxième organe de blocage (25) présente une crémaillère (26) qui est tournée vers le premier organe de blocage (35) et qui est réalisée de préférence sous forme d'arc de cercle.

7. Poignée d'instrument chirurgical selon l'une quelconque des revendications 1 à 6, dans laquelle le premier organe de blocage (35) est précontraint au moyen d'un ressort à lame (42) vers le deuxième organe de blocage (25), qui est de préférence fixé au premier élément de poignée (10) et qui s'applique contre l'élément de blocage (30).

8. Poignée d'instrument chirurgical selon l'une quelconque des revendications 1 à 7, dans laquelle l'appui pour le pouce (33, 33A) s'étend des deux côtés de l'axe longitudinal du premier élément de poignée (10) afin de pouvoir être actionné par la main droite et par la main gauche.

9. Instrument chirurgical comprenant
un composant de tige (1),
un organe d'actionnement (2),
une partie de mâchoire qui peut être actionnée par un mouvement axial relatif du composant de tige (1) et de l'organe d'actionnement (2),
**caractérisé par**
une poignée d'instrument chirurgical selon l'une quelconque des revendications précédentes.

10. Instrument chirurgical selon la revendication 9 dans lequel
le premier élément de poignée (10) est connecté rigidement à l'un parmi un composant de tige (1) ou un organe d'actionnement (2) d'un instrument chirurgical, et
le deuxième élément de poignée (20) est accouplé à l'autre parmi le composant de tige (1) ou l'organe d'actionnement (2) de l'instrument chirurgical.
